(19) Europäisches Patentamt
European Patent Office
Office européen des brevets

(11) **EP 2 204 165 A1**

(12) **DEMANDE DE BREVET EUROPEEN**

(43) Date de publication:
**07.07.2010 Bulletin 2010/27**

(21) Numéro de dépôt: **09181021.8**

(22) Date de dépôt: **30.12.2009**

(51) Int Cl.:
*A61K 8/60* *(2006.01)*      *A61Q 19/08* *(2006.01)*
*A61K 8/35* *(2006.01)*      *A61K 8/37* *(2006.01)*
*A61K 8/40* *(2006.01)*      *A61K 8/46* *(2006.01)*

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **30.12.2008 FR 0859146**
**15.01.2009 US 144757 P**

(71) Demandeur: **L'Oréal**
**75008 Paris (FR)**

(72) Inventeurs:
• **Laboureau, Julien**
**92130, Issy les Moulineaux (FR)**
• **Simonnet, Jean-Thierry**
**94230, Cachan (FR)**
• **Portes, Pascal**
**94130, Nogent sur Marne (FR)**

(74) Mandataire: **Tezier Herman, Béatrice et al**
**Cabinet Becker & Associés**
**25, rue Louis Le Grand**
**75002 Paris (FR)**

(54) **Association de monosaccharides avec des filtres solaires et son utilisation en cosmétique**

(57) La présente invention concerne une composition, notamment cosmétique et/ou dermatologique, comprenant dans un milieu physiologiquement acceptable, l'association d'au moins un monosaccharide choisi parmi le mannose, le rhamnose et leur mélange, et d'au moins un filtre solaire. La présente invention concerne également l'utilisation d'une telle composition.

EP 2 204 165 A1

**Description**

La présente invention concerne une composition, notamment cosmétique et/ou dermatologique, comprenant dans un milieu physiologiquement acceptable, l'association d'au moins un monosaccharide choisi parmi le mannose, le rhamnose et leur mélange, et d'au moins un filtre solaire. La présente invention concerne également l'utilisation d'une telle composition.

La peau humaine est constituée de deux couches principales qui sont le derme et l'épiderme qui recouvre le derme de manière superficielle. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures (climat, rayons ultraviolets, tabac...), appelé « fonction barrière ».

L'épiderme est un épithélium pavimenteux stratifié kératinisé constitué à 90% de kératinocytes. La différenciation progressive des cellules de la membrane basale, qui sépare le derme de l'épiderme, vers la surface de l'épiderme comprend notamment la différenciation des kératinocytes qui migrent vers la surface de la peau où ils desquament.

Le vieillissement de l'épiderme se manifeste principalement par la réduction de son épaisseur. L'atrophie de l'épiderme est la conséquence du ralentissement de la prolifération des kératinocytes et de l'accumulation de kératinocytes sénescents. La couche cornée devient terne.

La desquamation est un phénomène naturel lié au fait que l'épiderme, qui constitue la couche supérieure de la peau, est en constante régénération. L'épiderme est constitué de plusieurs assises de cellules, dont la plus profonde est l'assise basale constituée de cellules indifférenciées. Au cours du temps, ces cellules vont différencier et migrer vers la surface de l'épiderme en constituant les différentes assises de celui-ci, jusqu'à former à la surface de l'épiderme les cornéocytes qui sont des cellules mortes qui s'éliminent par desquamation. Cette perte en surface est compensée par la migration de cellules de l'assise basale vers la surface de l'épiderme. Il s'agit du renouvellement perpétuel de la peau. Une élimination forcée de la couche cornée accélère le renouvellement et permet de lutter contre le vieillissement.

Dans le même temps ces cellules poursuivent leur différenciation dont le dernier stade est le cornéocyte. Il s'agit en fait de cellules mortes qui constituent la dernière couche de l'épiderme, c'est à dire la couche la plus externe encore appelée stratum corneum.

Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes. La cohésion entre l'épiderme et le derme est assurée par la jonction dermo-épidermique. C'est une région complexe d'une épaisseur de 100 nm environ qui comprend le pole basal des kératinocytes basaux, la membrane épidermique et la zone sous-basale du derme superficiel.

Les collagènes sont les protéines majoritaires des matrices extracellulaires de la peau. A ce jour, 20 types de collagènes sont identifiés et notés de I à XX. Les collagènes majoritairement présents dans l'ensemble du derme sont les collagènes de type I et III qui forment la matrice extracellulaire de l'ensemble du derme (ce sont ces collagènes qui constituent 70-80 % du poids sec du derme). Par ailleurs, les collagènes ne sont pas tous synthétisés par les mêmes types cellulaires, les collagènes de type I et III sont essentiellement produits par le fibroblaste dermique alors que le collagène de type VII est produit par deux catégories de cellules, les kératinocytes et les fibroblastes. La régulation de leur expression diffère d'un collagène à un autre, par exemple, les collagènes I et VII ne sont pas régulés de la même manière par certaines cytokines, en effet, le TNF alpha et la leukoréguline stimulent le collagène VII et régulent négativement le collagène I. Enfin, toutes les molécules de collagène sont des variantes d'un précurseur commun qui est la chaîne alpha du procollagène.

Avec le vieillissement, le collagène s'amincit et des rides apparaissent à la surface de la peau. Le vieillissement cutané est un mécanisme conditionné en partie par les caractéristiques génétiques.

Par ailleurs, certains facteurs d'environnement comme le tabagisme et l'exposition au rayonnement du soleil l'accélèrent. La peau a ainsi un aspect beaucoup plus âgé sur les zones exposées au soleil comme le dos des mains ou le visage. Ainsi, ces autres facteurs ont également un impact négatif sur le collagène naturel de la peau.

En conséquence, compte tenu du rôle important du collagène au niveau de l'intégrité de la peau et de sa résistance aux agressions externes de type mécaniques, la stimulation de synthèse de ces collagènes, et en particulier du collagène de type I, apparaît comme un moyen efficace pour pallier les signes du vieillissement cutané.

Au cours du vieillissement, la peau subit donc de nombreuses modifications et dégradations qui se traduisent, avec l'âge, par une altération du microrelief, par une flaccidité, une perte de souplesse cutanée, une apparition de rides et ridules, l'apparition de taches pigmentaires, une altération des propriétés mécaniques de la peau, notamment un manque d'élasticité de la peau, et une perte d'éclat du teint.

On comprend donc l'importance de pouvoir disposer de produits dont les effets visent à lutter contre les signes globaux du vieillissement, à régénérer le tissu de la peau via une augmentation de la prolifération des kératinocytes, une stimulation de la prolifération et/ou du métabolisme des fibroblastes, et notamment une stimulation de la synthèse des collagènes.

A cet égard, la demanderesse a découvert, de manière surprenante et inattendue, que l'association d'au moins un monosaccharide choisi parmi le mannose, le rhamnose et leur mélange, et d'au moins un filtre solaire, organique ou minérale, est capable d'augmenter le nombre de kératinocytes et/ou de fibroblastes, de stimuler le métabolisme des fibroblastes, et/ou de stimuler la synthèse des collagènes, en particulier la synthèse de procollagène de type 1; et ainsi de contrer les signes du vieillissement cutané, et en particulier l'atrophie épidermique et/ou dermique liée au vieillissement. De plus, on peut considérer que les filtres UV ont une action préventive dans le vieillissement alors que les monosaccharides de l'invention ont une action réparatrice, ce qui leur permet lorsqu'ils sont associés de lutter contre les signes globaux du vieillissement de la peau et de ses phanères.

La demanderesse a en effet démontré l'activation de la prolifération des kératinocytes et/ou des fibroblastes et la stimulation de la synthèse du procollagène I par le mannose ou le rhamnose. L'utilisation de compositions les contenant permet ainsi de contrer les signes du vieillissement cutané, et en particulier l'atrophie épidermique et/ou dermique liée au vieillissement.

L'utilisation de ces monosaccharides était restée jusqu'alors inconnue pour les effets biologiques directs exposés plus haut. La demande WO 2007/128939 mentionne toutefois une activité anti-âge obtenue par un effet biomécanique d'un agent tenseur en association avec des composés saccharidiques, qui permettent d'augmenter l'expression des mécanorécepteurs de cellules de la peau. Cette augmentation de l'expression des mécanorécepteurs est décrite comme augmentant la sensibilisation des cellules de la peau à répondre aux effets des tenseurs. De même, la demande française FR2900572 mentionne un procédé de soin cosmétique de la peau impliquant l'utilisation conjointe d'une composition comprenant un composé saccharidique permettant d'augmenter l'expression des mécanorécepteurs de cellules de la peau et d'un dispositif destiné à appliquer des contraintes mécaniques sur la peau, ce qui permet d'augmenter l'efficacité des contraintes mécaniques grâce à l'augmentation du nombre de mécanorécepteurs de cellules de la peau.

La demande de brevet US 2007/0025933 décrit une composition comprenant une base photoprotectrice, constituée de deux types de composants, et éventuellement un mélange de composants additionnels, tels que notamment des monosaccharides (comme le mannose, fructose et glucose) pour stabiliser ladite composition. Aucune activité propre aux monosaccharides sur la peau n'est mentionnée.

La demande WO 2005/063194 décrit une base galénique à très haute tolérance comprenant notamment du mannose ou du rhamnose. Il est spécifié qu'une telle base galénique ne peut fonctionner qu'en association avec un actif dont elle est seulement le véhicule. Les bases galéniques dermiques et/ou cosmétiques divulguées reposent essentiellement sur la présence des deux polyols que sont le mannitol et le xylitol.

La présente invention concerne donc une composition, notamment cosmétique et/ou dermatologique, comprenant l'association d'au moins un monosaccharide choisi parmi le mannose, le rhamnose et leur mélange avec au moins un filtre solaire.

De préférence, la composition selon l'invention ne comprend pas l'association de xylitol et de mannitol. Selon une autre alternative, la composition selon l'invention ne comprend pas d'agent tenseur. De manière encore plus préférentielle, la composition selon l'invention ne comprend pas d'agent tenseur et ne comprend pas l'association de xylitol et de mannitol.

Selon un mode préféré de réalisation, le monosaccharide est le rhamnose.

Le mannose est un hexose épimère en C2 du glucose. Le rhamnose (ou 6 deoxy mannose) constitue formellement le produit de désoxygénation du mannose en C6. Les monosaccharides selon l'invention sont sous la forme D ou L du mannose et/ou du rhamnose ou leur mélange, chaque forme pouvant elle-même être l'anomère alpha et/ou béta. Les formes préférées selon l'invention sont le D-mannose ou le L-rhamnose.

Le D-mannose est présent dans les végétaux en particulier certains fruits dont les airelles (canneberges), ou le bois dur (hêtre, bouleau). Le rhamnose est trouvé dans la nature sous forme L. Le D-mannose, ainsi que le L-rhamnose, sont commercialisés, par exemple, par la société Danisco Sweeteners® et Symrise. Dans la présente invention, le monosaccharide est plus spécifiquement sous forme de monomère.

Le ou les filtre(s) solaire(s) (ou filtres UV) de la composition selon l'invention sont des filtres solaires organiques, minéraux ou leur mélange.

Parmi les filtres solaires, on peut notamment citer les filtres suivants.

Filtres UV organiques

Les filtres organiques sont notamment choisis parmi les dérivés de dibenzoylméthane ; les anthranilates ; les dérivés cinnamiques ; les dérivés salicyliques ; les dérivés du camphre ; les dérivés de la benzophénone ; les dérivés de β,β-diphénylacrylate , les dérivés de triazine ; les dérivés de benzotriazole ; les dérivés de benzalmalonate, notamment ceux cités dans le brevet US5624663 ; les dérivés de benzimidazole ; les imidazolines ; les dérivés bis-benzoazolyle, tels que décrits dans les brevets EP669323 et US 2,463,264 ; les dérivés de l'acide p-aminobenzoïque (PABA) ; les dérivés de méthylène bis-(hydroxyphényl benzotriazole), tels que décrits dans les demandes US5,237,071, US 5,166,355, GB2303549, DE 197 26 184 et EP893119 ; les dérivés de benzoxazole, tels que décrits dans les demandes de brevet EP0832642, EP1027883, EP1300137 et DE10162844 ; les polymères filtres et silicones filtres, tels que ceux décrits notamment dans la demande WO-93/04665 ; les dimères dérivés d'α-alkylstyrène, tels que ceux décrits dans la demande de brevet DE19855649 ; les 4,4-diarylbutadiènes, tels que décrits dans les demandes EP0967200, DE19746654, DE19755649, EP-A-1008586, EP1133980 et EP133981 ; les dérivés de mérocyanines, tels que ceux décrits dans les demandes WO04006878, WO05058269 et WO06032741, et leurs mélanges.

De préférence, les filtres organiques sont choisis parmi les anthranilates ; les dérivés salicyliques , les dérivés de la benzophénone ; les dérivés de diphénylacrylate ; les dérivés de triazine ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes ; les dérivés de mérocyanines et leurs mélanges.

Comme exemples d'agents photoprotecteurs organiques, on peut citer ceux désignés ci-dessous sous leur nom INCI :

Dérivés de dibenzoylméthane:
Butylmethoxydibenzoylmethane, vendu notamment sous le nom commercial PARSOL MCX par DSM Nutritional Products, Inc,

Dérivés cinnamiques :
Ethylhexyl Methoxycinnamate, vendu notamment sous le nom commercial PARSOL MCX par DSM Nutritional Products, Inc,
Isopropyl Methoxycinnamate,
Isoamyl Methoxycinnamate, vendu notamment sous le nom commercial NEO HELIOPAN E 1000 par SYMRISE,
DEA Methoxycinnamate,
Diisopropyl Methylcinnamate, ou
Glyceryl Ethylhexanoate Dimethoxycinnamate.

Dérivés de l'acide oara-aminobenzoique :
PABA,
Ethyl PABA,
Ethyl Dihydroxypropyl PABA,
Ethylhexyl Diméthyl PABA, vendu notamment sous le nom « ESCALOL 507 » par ISP,
Glyceryl PABA,
PEG-25 PABA, vendu notamment sous le nom « UVINUL P25 » par BASF,

Dérivés salicyliques :
Homosalate, vendu notamment sous le nom « Eusolex HMS » par Rona/EM Industries,
Ethylhexyl Salicylate, vendu notamment sous le nom « NEO HELIOPAN OS » par SYMRISE,
Dipropyleneglycol Salicylate, vendu notamment sous le nom « DIPSAL » par SCHER,
TEA Salicylate, vendu notamment sous le nom « NEO HELIOPAN TS » par SYMRISE.

Dérivés de β,β-diphénylacrylate :
Octocrylene, vendu notamment sous le nom commercial « UVINUL N539 » par BASF,
Etocrylene, vendu notamment sous le nom commercial « UVINUL N35 » par BASF.

Dérivés de la benzophénone :
Benzophenone-1, vendu notamment sous le nom commercial « UVINUL 400 » par BASF,
Benzophenone-2, vendu notamment sous le nom commercial « UVINUL D50 » par BASF,
Benzophenone-3 ou Oxybenzone, vendu notamment sous le nom commercial « UVINUL M40 » par BASF,
Benzophenone-4, vendu notamment sous le nom commercial « UVINUL MS40 » par BASF,
Benzophenone-5,
Benzophenone-6, vendu notamment sous le nom commercial « Helisorb 11 » par Norquay,
Benzophenone-8, vendu notamment sous le nom commercial « Spectra-Sorb UV-24 » par American Cyanamid,
Benzophenone-9, vendu notamment sous le nom commercial« UVINUL DS-49» par BASF,
Benzophenone-12,
2-(4-diethylamino-2-hydroxybenzoyl)-benzoate de n-hexyle, vendu notamment sous le nom commercial « UVINUL A+ » par BASF.

Dérivés du benzylidène camphre :
3-Benzylidene camphor, fabriqué sous le nom « MEXORYL SD» par CHIMEX,
4-Methylbenzylidene camphor, vendu sous le nom « EUSOLEX 6300 » par MERCK,
Benzylidene Camphor Sulfonic Acid, fabriqué sous le nom « MEXORYL SL» par CHIMEX,
Camphor Benzalkonium Methosulfate, fabriqué sous le nom « MEXORYL SO » par CHIMEX,
Terephthalylidene Dicamphor Sulfonic Acid, fabriqué sous le nom « MEXORYL SX » par CHIMEX,
Polyacrylamidomethyl Benzylidene Camphor, fabriqué sous le nom « MEXORYL SW » par CHIMEX.

Dérivés du phenyl benzimidazole :
Phenylbenzimidazole Sulfonic Acid, vendu notamment sous le nom commercial « EUSOLEX 232 » par MERCK,
Disodium Phenyl Dibenzimidazole Tetra-sulfonate, vendu notamment sous le nom commercial « NEO HELIOPAN AP » par SYMRISE.

Dérivés du phenyl benzotriazole :
Drometrizole Trisiloxane, vendu notamment sous le nom « Silatrizole » par RHODIA CHIMIE
Methylène bis-Benzotriazolyl Tetramethylbutylphénol, vendu notamment sous forme solide sous le nom commercial « MIXXIM BB/100 » par FAIRMOUNT CHEMICAL ou sous forme micronisée en dispersion aqueuse sous le nom commercial « TINOSORB M » par CIBA SPECIALTY CHEMICALS.

Dérivés de triazine :

- Bis-Ethylhexyloxyphenol Methoxyphenyl Triazine, vendu notamment sous le nom commercial «TINOSORB S » par CIBA GEIGY,
- Ethylhexyl triazone, vendu notamment sous le nom commercial «UVINUL T150 » par BASF,
- Diethylhexyl Butamido Triazone, vendu sous le nom commercial « UVASORB HEB » par SIGMA 3V,
- la 2,4,6-tris(4'-amino benzalmalonate de dinéopentyle)-s-triazine,
- la 2,4,6-tris-(4'-amino benzalmalonate de diisobutyle)-s- triazine,
- la 2,4bis (4'-aminobenzoate de n-butyle)-6-(aminopropyltrisiloxane)-s-triazine,
- la 2,4bis (4'-aminobenzalmalonate de dinéopentyle)-6-(4'-aminobenzoate de n-butyle)-s-triazine,
- les filtres triazines symétriques décrits dans le brevet US6,225,467, la demande WO2004/085412 (voir composés 6 et 9) ou le document « Symetrical Triazine Derivatives » IP.COM Journal , IP.COM INC WEST HENRIETTA, NY, US (20 septembre 2004) notamment les 2,4,6-tris-(biphényl)-1,3,5-triazines (en particulier la 2,4,6-tris(biphenyl-4-yl-1,3,5-triazine et la 2,4,6-tris(terphenyl)-1,3,5-triazine qui est repris dans les demandes de brevet WO06/035000, WO06/034982, WO06/034991, WO06/035007, WO2006/034992, WO2006/034985.

Dérivés anthraniliques :
Menthyl anthranilate, vendu notamment sous le nom commercial « NEO HELIOPAN MA » par SYMRISE,

Dérivés d'imidazolines :
Ethylhexyl Dimethoxybenzylidene Dioxoimidazoline Propionate,

Dérivés du benzalmalonate :
Polyorganosiloxane à fonctions benzalmalonate comme le Polysilicone-15 vendu sous la dénomination commerciale « PARSOL SLX » par DSM Nutritional Products, Inc,

Dérivés de 4,4-diarylbutadiène :
-1,1-dicarboxy (2,2'-diméthyl-propyl)-4,4-diphénylbutadiène,

Dérivés de benzoxazole :
2,4-bis-[5-1(diméthylpropyl)benzoxazol-2-yl-(4-phenyl)-imino]-6-(2-ethylhexyl)-imino-1,3,5-triazine, vendu notamment sous le nom d'Uvasorb K2A par Sigma 3V
et leurs mélanges.

Dérivés de mérocyanine
Octyl-5-N,N-diethylamino-2-phenysulfonyl-2,4-pentadienoate

Les filtres organiques préférentiels sont choisis parmi :

Ethylhexyl Methoxycinnamate,
Ethylhexyl Salicylate,
Homosalate,
Octocrylene,
Phenylbenzimidazole Sulfonic Acid,
Benzophenone-3,
2-(4-diethylamino-2-hydroxybenzoyl)-benzoate de n-hexyle.
Terephthalylidene Dicamphor Sulfonic Acid,
Methylène bis-Benzotriazolyl Tetramethylbutylphénol,
Bis-Ethylhexyloxyphenol Methoxyphenyl Triazine,
Diethylhexyl Butamido Triazone,
la 2,4,6-tris(4'-amino benzalmalonate de dinéopentyle)-s-triazine,
la 2,4,6-tris-(4'-amino benzalmalonate de diisobutyle)-s-triazine,
la 2,4-bis-(4'-aminobenzoate de n-butyle)-6-(aminopropyltrisiloxane)-s-triazine,
la 2,4-bis-(4'-aminobenzalmalonate de dinéopentyle)-6-(4'-aminobenzoate de n-butyle)-s-triazine,
Drometrizole Trisiloxane,
Octyl-5-N,N-diethylamino-2-phenysulfonyl-2,4-pentadienoate,

et leurs mélanges.

Filtres minéraux :

Les filtres inorganiques sont choisis parmi des pigments d'oxydes métalliques enrobés ou non, dont la taille moyenne des particules primaires est préférentiellement comprise entre 5 nm et 100 nm (de préférence entre 10 nm et 50 nm), comme par exemple des pigments d'oxyde de titane (amorphe ou cristallisé sous forme rutile et/ou anatase), de fer, de zinc, de zirconium ou de cérium ou leurs mélanges qui sont tous des agents photoprotecteurs UV bien connus en soi.

Les pigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

De façon connue, les silicones sont des polymères ou oligomères organo-siliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium.

Le terme "silicones" englobe également les silanes nécessaires à leur préparation, en particulier, les alkyl silanes.

Les silicones utilisées pour l'enrobage des pigments convenant à la présente invention sont de préférence choisies dans le groupe contenant les alkyl silanes, les polydialkylsiloxanes, et les polyalkylhydrogénosiloxanes. Plus préférentiellement encore, les silicones sont choisies dans le groupe contenant l'octyl triméthyl silane, les polydiméthylsiloxanes et les polyméthylhydrogénosiloxanes.

Bien entendu, les pigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Les pigments enrobés sont plus particulièrement des oxydes de titane enrobés :

- de silice, tels que le produit "SUNVEIL" de la société IKEDA et le produit " Eusolex T-AVO" de la société MERCK
- de silice et d'oxyde de fer, tels que le produit "SUNVEIL F" de la société IKEDA,
- de silice et d'alumine, tels que les produits "MICROTITANIUM DIOXIDE MT 500 SA" et "MICROTITANIUM DIOXIDE MT 100 SA" de la société TAYCA, "TIOVEIL" de la société TIOXIDE, et « Mirasun TiW 60 » de la société Rhodia,
- d'alumine, tels que les produits "TIPAQUE TTO-55 (B)" et "TIPAQUE TTO-55 (A)" de la société ISHIHARA, et "UVT 14/4" de la société KEMIRA,

- d'alumine et de stéarate d'aluminium, tels que le produit "MICROTITANIUM DIOXIDE MT 100 TV, MT 100 TX, MT 100 Z, MT-01 de la société TAYCA, les produits "Solaveil CT-10 W", "Solaveil CT 100" et "Solaveil CT 200" de la société UNIQEMA,
- de silice, d'alumine et d'acide alginique, tel que le produit " MT-100 AQ" de la société TAYCA,
- d'alumine et de laurate d'aluminium, tel que le produit "MICROTITANIUM DIOXIDE MT 100 S" de la société TAYCA,
- d'oxyde de fer et de stéarate de fer, tels que le produit "MICROTITANIUM DIOXIDE MT 100 F" de la société TAYCA,
- d'oxyde de zinc et de stéarate de zinc, tels que le produit "BR351" de la société TAYCA,
- de silice et d'alumine et traités par une silicone, tels que les produits "MICROTITANIUM DIOXIDE MT 600 SAS", "MICROTITANIUM DIOXIDE MT 500 SAS" ou "MICROTITANIUM DIOXIDE MT 100 SAS" de la société TAYCA,
- de silice, d'alumine, de stéarate d'aluminium et traités par une silicone, tels que le produit "STT-30-DS" de la société TITAN KOGYO,
- de silice et traité par une silicone, tel que le produit "UV-TITAN X 195" de la société KEMIRA, ou le produit SMT-100 WRS de la société TAYCA.
- d'alumine et traités par une silicone, tels que les produits "TIPAQUE TTO-55 (S)" de la société ISHIHARA, ou "UV TITAN M 262" de la société KEMIRA,
- de triéthanolamine, tels que le produit "STT-65-S" de la société TITAN KOGYO,
- d'acide stéarique, tels que le produit "TIPAQUE TTO-55 (C)" de la société ISHIHARA,
- d'hexamétaphosphate de sodium, tels que le produit "MICROTITANIUM DIOXIDE MT 150 W" de la société TAYCA.

D'autres pigments d'oxyde de titane traités avec une silicone sont de préférence le TiO2 traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO2 traité par un polydiméthylsiloxane et dont la taille moyenne des particules élémentaires est de 21 nm tel que celui vendu sous la dénomination commerciale "70250 Cardre UF TiO2SI3" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les pigments d'oxyde de zinc non enrobés, sont par exemple ceux commercialisés sous la dénomination "Z-cote" par la société Sunsmart.

Les pigments d'oxyde de zinc enrobés sont par exemple :

- ceux commercialisés sous la dénomination « Z-COTE HP1 » par la société SUNSMART (ZnO enrobé dimethicone) ;
- ceux commercialisés sous la dénomination "Oxide zinc CS-5" par la société Toshibi (ZnO enrobé par polymethylhydrogenesiloxane) ;
- ceux commercialisés sous la dénomination "DAITOPERSION ZN-30" et "DAITOPERSION Zn-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD Ultrafine ZnO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "Escalol Z100" par la société ISP (ZnO traité alumine et dispersé dans le mélange methoxycinnamate d'ethylhexyle / copolymère PVP-hexadecene / methicone) ;
- ceux commercialisés sous la dénomination "Fuji ZnO-SMS-10" par la société Fuji Pigment (ZnO enrobé silice et polymethylsilsesquioxane).

Les pigments d'oxyde de cérium non enrobé sont vendus par exemple sous la dénomination "COLLOIDAL CERIUM OXIDE" par la société RHONE POULENC.

Les pigments d'oxyde de fer enrobés sont par exemple vendus par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

La présente invention porte aussi sur l'utilisation, notamment cosmétique ou dermatologique, d'une composition ou d'une association selon l'invention telle que définie précédemment, pour le soin de la peau et/ou du cuir chevelu, de préférence administrée par voie topique.

Une composition conforme à l'invention telle que définie précédemment peut notamment être une composition cosmétique pour soin capillaire pour, en particulier, la stimulation de la pousse du cheveu, la lutte contre la chute des cheveux, le ralentissement de sa chute ou le renforcement de l'éclat du cheveu.

La présente invention a également pour objet une méthode de traitement, en particulier cosmétique ou thérapeutique, pour diminuer ou prévenir les signes du vieillissement de la peau ou de ses phanères (cheveux, cils, ongles, ...), par administration à un sujet, de préférence un être humain, d'une quantité efficace d'au moins un monosaccharide tel que défini précédemment en association avec une quantité efficace d'au moins un filtre solaire tel que défini précédemment.

La présente invention concerne également l'utilisation, notamment cosmétique ou dermatologique, de la composition ou de l'association selon l'invention, pour diminuer et/ou prévenir les signes du vieillissement de la peau ou de ses phanères (cheveux, cils, ongles,...), en particulier pour diminuer ou prévenir les signes du chronovieillissement de la peau ou de ses phanères.

Selon un mode particulier, la composition utilisée dans la cadre de la présente invention ne comprend pas l'association de xylitol et de mannitol.

La composition ou l'association selon l'invention permet aussi de stimuler la régénération des cellules de l'épiderme et du derme, au niveau de la peau ou des phanères, en particulier les kératinocytes et les fibroblastes, notamment par augmentation de leur prolifération. On dispose de la sorte d'une méthode, notamment cosmétique, efficace notamment pour lutter contre les signes du vieillissement.

Les signes du chronovieillissement correspondent aux dégradations internes de la peau dues au vieillissement intrinsèque des individus. Les signes du photovieillissement correspondent aux dégradations internes de la peau consécutives à une exposition aux rayonnements ultra-violets (vieillissement actinique), l'association selon l'invention et en particulier les filtres solaires contenus dans l'association permet également de lutter efficacement contre ces signes de vieillissement.

Selon une forme de réalisation préférée, l'utilisation selon la présente invention est destinée à améliorer l'éclat du teint, à diminuer et/ou prévenir les caractéristiques des rides et/ou ridules, à diminuer et/ou prévenir les taches pigmentaires, à améliorer et/ou diminuer le micro-relief de la peau, à lisser la peau et/ou à améliorer les propriétés mécaniques de la peau (notamment élasticité et/ou tonus de la peau) et/ou favoriser la réparation de la peau.

Selon un autre aspect de l'invention, l'utilisation de la composition ou de l'association selon l'invention permet d'améliorer la densité de la peau ou sa fermeté.

La présente invention concerne également l'utilisation de la composition ou de l'association selon l'invention pour traiter, de manière préventive ou curative, les rides et/ou ridules, la peau flétrie, le manque d'élasticité et/ou de tonus de la peau, l'amincissement du derme, la dégradation des fibres de collagène, la peau molle et/ou la peau amincie.

La composition ou l'association selon la présente invention a également pour effet d'augmenter la synthèse des collagènes, de préférence le pro-collagène I.

La quantité des ingrédients actifs, choisis parmi les monosaccharides et les filtres solaires, à mettre en oeuvre selon l'invention dépend de l'effet cosmétique ou thérapeutique recherché, et peut donc varier dans une large mesure. L'homme de l'art peut aisément, sur la base de ses connaissances générales, déterminer les quantités appropriées.

Ainsi, et selon une forme de réalisation préférée, la composition selon l'invention comprend au moins un monosaccharide tel que défini ci-dessus en une quantité comprise entre 0,001 % et 30 % en poids par rapport au poids total de la composition, et en particulier entre 0,1 % et 10 % en poids, et plus particulièrement entre 0,5 % et 6 % en poids par rapport au poids total de la composition.

Selon une autre forme de réalisation préférée, la composition selon l'invention comprend au moins un monosaccharide tel que défini ci-dessus en une quantité comprise entre 0,3 % et 10 % en poids par rapport au poids total de la composition.

Selon une forme de réalisation préférée, la composition selon l'invention comprend au moins un filtre solaire en une quantité comprise entre 0,01 % et 20 % en poids par rapport au poids total de la composition, et en particulier entre 0,1 % et 10 % en poids par rapport au poids total de la composition.

Selon une forme de réalisation préférée, la composition selon l'invention comprend au moins un filtre solaire en une quantité comprise entre 0,1 % et 30 % en poids par rapport au poids total de la composition, et en particulier entre 1 % et 20 % en poids par rapport au poids total de la composition.

Selon une forme de réalisation hautement préférée, la composition selon l'invention comprend au moins un monosaccharide tel que défini ci-avant en une quantité comprise entre 0,3 % et 10 % et au moins un filtre solaire en une quantité comprise entre 0,1 % et 20 % en poids par rapport au poids total de la composition.

La composition selon l'invention est de préférence adaptée à une administration topique sur la peau ou ses phanères.

De préférence, les administrations topiques selon l'invention se présentent sous forme d'une crème, d'un gel, d'une lotion, d'un lait, d'une huile, d'un onguent, d'une cire, d'une mousse, d'une pâte, d'un sérum, d'une pommade ou d'un shampooing.

Le monosaccharide selon l'invention et le filtre solaire sont plus particulièrement présents dans la composition selon l'invention à titre d'agent (ou ingrédient) actif, en particulier de seuls agents actifs.

Par « agent actif » ou « ingrédient actif », on entend plus spécifiquement selon l'invention, un composé qui, lorsqu'il est administré à un sujet, en particulier un sujet humain, joue un rôle biologique direct sur l'organisme, en particulier sur la peau ou ses phanères, en particulier sans améliorer l'effet biologique ou mécanique d'un autre composé présent dans la composition selon l'invention.

Plus particulièrement, la composition selon l'invention ne comprend pas de monosaccharide additionnel.

De manière générale on entend par « agent tenseur » tous composés solubles ou dispersibles dans l'eau à une température allant de 25°C à 50°C à la concentration de 7% en poids dans l'eau ou à la concentration maximale à laquelle ils forment un milieu d'apparence homogène et produisant à cette concentration de 7% ou à cette concentration maximale dans l'eau une rétractation de plus de 15 % dans le test décrit ci-après.

La concentration maximale à laquelle ils forment un milieu d'apparence homogène est déterminée à $\pm$ 20% près et de préférence à $\pm$ 5% près.

On entend par « milieu d'apparence homogène » un milieu ne présentant pas d'agrégats visibles à l'oeil nu.

Pour la détermination de ladite concentration maximale, l'agent tenseur est ajouté progressivement dans l'eau sous agitation à la défloculeuse à une température allant de 25°C à 50°C, puis le mélange est maintenu sous agitation pendant une heure. On observe ensuite après 24 heures si le mélange ainsi préparé est d'apparence homogène (absence d'agrégats visibles à l'oeil nu).

L'effet tenseur peut être caractérisé par un test in vitro de rétractation.

On prépare préalablement et tel que décrit précédemment un mélange homogène de l'agent tenseur dans l'eau, à la concentration de 7% en poids ou à la concentration maximale définie précédemment.

$30\mu$l du mélange homogène est déposé sur une éprouvette rectangulaire (10x40mm, donc présentant une largeur initiale $L_0$ de 10 mm) d'élastomère ayant un module d'élasticité de 20MPa et une épaisseur de $100\mu$m.

Après 3h de séchage à $22\pm3$°C et $40\pm10$% d'humidité relative HR, l'éprouvette d'élastomère présente une largeur rétractable, notée $L_{3h}$ due à la tension exercée par l'agent tenseur déposé.

L'effet tenseur (ET) dudit agent est alors qualifié de la façon suivante :

$$'ET' = (L_0 - L_{3h} / L_0) \times 100 \text{ en } \%$$

$$\text{avec } L_0 = \text{largeur initiale 10mm}$$
$$\text{et } L_{3h} = \text{largeur après 3h de séchage}$$

L'agent tenseur peut être choisi parmi :

a) les protéines végétales ou animales et leurs hydrolysats ;
b) les polysaccharides d'origine naturelle ;
c) les silicates mixtes ;
d) les particules colloïdales de charges inorganiques ;
e) les polymères synthétiques ;

et les mélanges de ceux-ci.

L'homme du métier saura choisir, dans les catégories chimiques listées ci-dessus, les matériaux répondant au test tel que décrit précédemment.

D'une manière générale, le milieu dans lequel sont compris les principes actifs de la composition tel que définie précédemment, est un milieu physiologiquement acceptable, en particulier un milieu cosmétiquement ou pharmaceutiquement acceptable et peut être anhydre ou aqueux. Il peut ainsi comprendre une phase aqueuse et/ou une phase grasse.

Le milieu physiologiquement acceptable dans lequel les composés selon l'invention peuvent être employés, ainsi que ses constituants, leur quantité, la forme galénique de la composition, son mode de préparation, et son mode d'administration peuvent être choisis par l'homme du métier sur la base de ses connaissances générales en fonction du type de composition recherchée.

Lorsque la composition est une composition destinée à une administration topique, elle peut se présenter avantageusement sous la forme de solutions aqueuses, hydroalcooliques, d'émulsions huile-dans-eau (H/E) ou eau-dans-huile (E/H) ou multiple (triple : E/H/E ou H/E/H), des nanoémulsions, en particulier des nanoémulsions H/E, dont la tailles des gouttes est inférieure à 100nm, de gels aqueux, ou de dispersions d'une phase grasse dans une phase aqueuse à l'aide de sphérules, ces sphérules pouvant être des nanoparticules polymériques telles que les nanosphères et les nanocapsules ou des vésicules lipidiques de type ionique et/ou non ionique (liposomes, niosomes, oléosomes (tels que décrits dans les demandes FR2709666 et FR2725369)).

Ces compositions sont préparées selon les méthodes usuelles.

En outre, les compositions utilisables selon l'invention peuvent être plus ou moins fluides et avoir l'aspect d'une crème blanche ou colorée, d'une pommade, d'un lait, d'une lotion, d'un sérum, d'une pâte ou d'une mousse. Elles peuvent être éventuellement appliquées sur la peau sous forme d'aérosol. Elles peuvent aussi se présenter sous forme solide, et par exemple sous forme de stick.

Pour une application locale sur les cheveux ou le cuir chevelu, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.

Lorsque la composition est une émulsion, la proportion de la phase grasse peut varier d'environ 5 % à 80 % en poids, et de préférence d'environ 2 % à 50 % en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 % à 30 % en poids, et de préférence de 0,5 % à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

La phase huileuse peut comprendre aussi tout additif habituel liposoluble ou lipodispersible comme indiqué ci-après.

Elle peut notamment comprendre des corps gras tels que des cires, des composés pâteux, des alcools gras, des acides gras. La phase huileuse contient au moins une huile, plus particulièrement au moins une huile cosmétique. On entend par "huile" un corps gras liquide à la température ambiante (25°C).

Comme huiles utilisables dans la composition de l'invention, on peut citer par exemple :

- les huiles hydrocarbonées d'origine animale, telles que le perhydrosqualène;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple, les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de jojoba, l'huile de beurre de karité, le caprylyl glycol ;
- les esters et les éthers de synthèse, notamment d'acides gras, comme les huiles de formules $R_1 COOR_2$ et $R_1OR_2$ dans laquelle $R_1$ représente le reste d'un acide gras ou d'unalcool gras comportant de 8 à 29 atomes de carbone, et $R_2$ représente une chaîne hydrocarbonée, ramifiée ou non, contenant de 3 à 30 atomes de carbone, comme par exemple l'huile de Purcellin, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle, l'isopropyl lauroyl sarcosinate, notamment vendu sous le nom commercial Eldew SL 205 de la société Ajinomoto ;
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam, le mélange de n-undécane (C11) et de n-tridécane (C13) commercialisé sous la référence de CETIOL UT par la Société Cognis ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles, en particulier cyclopolydiméthylsiloxanes (cyclométhicones) telles que le cyclohexadiméthylsiloxane et le cyclopentadiméthylsiloxane ; lespolydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;

- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination « Dow Corning 5200 Formulation Aid » par la société Dow Corning et le Cetyl dimethicone copolyol vendu sous la dénomination « Abil EM 90® » par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

Ces compositions peuvent être également des émulsions H/E stabilisées par des particules comme par exemple des particules polymériques décrites dans le brevet FR2760641, des polymères amphiphiles réticulés ou non tels que décrits dans les demandes : FR2853543 et FR2819175.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les absorbeurs d'odeurs et les matières colorantes. Les quantités de ces différents adjuvants sont celles classiquement utilisées dans le domaine cosmétique, et par exemple varient d'environ 0,01 % à 10 % du poids total de la composition. Ces adjuvants, selon leur nature, peuvent être introduits dans la phase grasse, dans la phase aqueuse et/ou dans les sphérules lipidiques.

Comme solvants utilisables dans l'invention, on peut citer les alcools inférieurs notamment l'éthanol, l'isopropanol, le dipropylène glycol, le butylène glycol et le propylène glycol.

Comme gélifiants hydrophiles utilisables dans l'invention, on peut citer à titre d'exemples non limitatifs, les polymères carboxyvinyliques (carbomer®), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, l'éthylcellulose et le polyéthylène.

Lorsque la composition est administrée par voie orale, elle est avantageusement sous la forme d'une gélule, d'un comprimé, ou de pilules. Lorsque la composition est administrée par injection cutanée, elle est en particulier sous la forme d'une solution stérile.

Les compositions de l'invention peuvent contenir d'autres actifs hydrophiles ou lipophiles. Ces actifs sont notamment choisis parmi les agents anti-oxydants, les agents dermo-relaxants ou dermodécontractants, les agents anti-âge, les agents anti-glycation, les agents stimulants la synthèse de macromolécules dermiques ou épidermiques et/ou empêchant leur dégradation, les agents stimulants la prolifération des fibroblastes ou des kératinocytes et/ou la différenciation des kératinocytes, les agents favorisant la maturation de l'enveloppe cornée, les inhibiteurs de NO synthase et les agents stimulant le métabolisme énergétique des cellules. Des listes de ces actifs sont données à la suite à titre illustratif, et ne doivent en aucune façon être considérées comme limitatives.

Agents anti-âge

Parmi les actifs connus pour lutter contre les signes du vieillissement, notamment cutané, on peut citer notamment :

la vitamine B3, le coenzyme Q10 (ou ubiquinone), la vitamine B9, la vitamine E, les dérivés de la vitamine E tels que le dérivé phosphaté comme par exemple le TPNA® commercialisé par la société Showa Denko, le resvératrol ou ses dérivés, comme par exemple le resveratrate® commercialisé par la société Estée Lauder, le rétinol ou ses dérivés, et leur mélange.

Agents anti-qlvcation:

Par « agent anti-glycation », on entend un composé prévenant et/ou diminuant la glycation des protéines de la peau, en particulier des protéines du derme, telles que le collagène.

Comme agents anti-glycation, on peut citer notamment les extraits végétaux de la famille des *Ericaceae,* tels qu'un extrait de myrtille (*Vaccinium angusfifollium, Vaccinium myrtillus*), par exemple celui vendu sous la dénomination « BLUEBERRY HERBASOL EXTRACT PG » par la société COSMETOCHEM, l'ergothionéine et ses dérivés, les hydroxystilbènes et leurs dérivés, tels que le resvératrol et le 3,3', 5,5'-tétrahydroxystilbène (ces agents anti-glycation sont décrits dans les demandes FR 2 802 425, FR 2 810 548, FR 2 796 278 et FR 2 802 420, respectivement), les dihydroxystilbènes et leurs dérivés, les polypeptides d'arginine et de lysine tels que celui vendu sous la dénomination « AMADORINE® » par la société SOLABIA, le chorhydrate de carcinine (commercialisé par Exsymol sous la dénomination « ALISTIN®»), un extrait d'*Hélianthus annuus* comme l'Antiglyskin® de SILAB, les extraits de vin tel que l'extrait de vin blanc en poudre sur support maltodextrine vendu sous la dénomination « Vin blanc déshydraté 2F » par la société Givaudan, l'acide thioctique (ou acide alpha lipoïque), un mélange d'extrait de busserole et de glycogène marin comme l'Aglycal LS 8777® de Laboratoires Sériobiologiques, un extrait de thé noir comme le Kombuchka® de Sederma et leurs mélanges.

Comme agents anti-glycation préférés, on citera les extraits de myrtille (*Vaccinium myrtillus*) et l'extrait de thé noir.

Agents stimulant la synthèse de macromolécules dermiques et/ou épidermiques et/ou empêchant leur dégradation :

Parmi les actifs stimulant les macromolécules du derme ou empêchant leur dégradation, on peut citer ceux qui agissent :

- soit sur la synthèse du collagène, tels que les extraits de Centella asiatica, les asiaticosides et dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ; les hormones végétales telles que les auxines et les lignanes ; l'acide folique ; et un extrait de Medicago sativa (alfafa) tel que celui commercialisé par SILAB sous la dénomination Vitanol®; un extrait peptidique de noisette tel que celui commercialisé par la société Solabia sous la dénomination Nuteline C®; et l'arginine.

- soit sur l'inhibition de la dégradation du collagène, en particulier des agents agissant sur l'inhibition des métalloprotéinases (MMP) telles que plus particulièrement les MMP 1, 2, 3, 9 . On peut citer : les rétinoïdes et dérivés, les extraits de medicago sativa tels que le Vitanol® de Silab, un extrait d'aphanizomenon flos-aquae (cyanophycée) commercialisée sous la dénomination Lanablue® par Atrium Biotechnologies, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon; un extrait de litchi ; la DIPALMITOYL HYDROXYPROLINE commercialisé par Seppic sous le nom SEPILIFT DPHP® : Baccharis genistelloide ou Baccharine commercialisé par SILAB, un extrait de moringa tel que l'Arganyl LS 9781® de Cognis ; l'extrait de sauge décrit dans la demande FR-A-2812544 de la famille des labiées (*salvia officinalis* de la société Flacksmann), l'extrait de Rhododendron, le blueberry extract, un extrait de *vaccinium myrtillus* tel que ceux décrits dans la demande FR-A-2814950.

- soit sur la synthèse de molécules appartenant à la famille des élastines (élastine et fibrilline), tels que : le rétinol et dérivés en particulier le palmitate de rétinol ; l'extrait de *Saccharomyces Cerivisiae* commercialisé par la société LSN sous la dénomination commerciale Cytovitin® ; et l'extrait d'algue *Macrocystis pyrifera* commercialisé par la société SECMA sous la dénomination commerciale Kelpadelie®; un extrait peptidique de noisette tel que celui commercialisé par la société Solabia sous la dénomination Nuteline C®.

- soit sur l'inhibition de la dégradation de l'élastine tels que l'extrait peptidique de graines de *Pisum sativum* commercialisé par la société LSN sous la dénomination commerciale Parelastyl® ; les héparinoïdes ; et les composés N-acylaminoamides décrits dans la demande WO 01/94381 tels que l'acide 12-[acétyl-(3-trifluorométhyl-phényl)-amino]-3-méthyl-butyrylaminol acétique, autrement nommé N-[N-acétyl, N'-(3-trifluorométhyl)phényl valyl]glycine ou N-acetyl-N-[3-(trifluoromethyl)phenyl]valyl-glycine ou acetyl trifluoromethyl phenyl valylglycine, ou un ester de celui-ci avec un alcool en $C_1$-$C_6$. un extrait de peptides de riz tel que la Colhibin® de Pentapharm, ou un extrait de Phyllanthus emblica tel que l'Emblica® de Rona.

- soit sur la synthèse des glycosaminoglycannes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; l'extrait de *Saccharomyces cerevisiae* disponible notamment auprès de la société SILAB sous la dénomination commerciale Firmalift® ou auprès de la société LSN sous la dénomination commerciale Cytovitin® ; un extrait de *Laminaria ochroieuca* tel que la Laminaïne® de Secma ; l'essence de Mamaku de Lucas Meyer, un extrait de cresson (Odraline® de Silab).

- soit sur la synthèse de la fibronectine, tels que l'extrait de zooplancton Salina commercialisé par la société SEPORGA sous la dénomination commerciale GP4G® ;
l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil®.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre *Fagus sylvatica* commercialisé par la société GATTEFOSSE sous la dénomination commerciale Gatuline® RC; et l'extrait de zooplancton Salina commercialisé par la société SEPORGA sous la dénomination commerciale GP4G®; Tripeptide de Cuivre de PROCYTE ; un extrait peptidique de *Voandzeia substerranea* tel que celui commercialisé par la société Laboratoires Sérobiologiques sous la dénomination commerciale Filladyn LS 9397®.

De préférence, on utilisera un actif stimulant la synthèse de macromolécules dermiques et/ou épidermiques et/ou empêchant leur dégradation choisi parmi les agents stimulant la synthèse des glycosaminoglycanes, les agents inhibant la dégradation de l'élastine, les agents stimulant la synthèse de la fibronectine, les agents stimulant la synthèse de macromolécules épidermiques, et leurs mélanges.

Encore plus préférentiellement, on utilisera un actif stimulant la synthèse des glycosaminoglycanes choisi parmi un extrait d'algue brune Padina pavonica, un extrait de *Saccharomyces cerevisiae,* un extrait de *Laminaria ochroleuca*, l'essence de Mamaku, un extrait de cresson et leurs mélanges.

Comme actifs préférés stimulant la synthèse de macromolécules dermiques et/ou épidermiques et/ou empêchant leur dégradation, on peut citer :

les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ; l'acide folique ; un extrait de Medicago sativa (alfafa) tel que celui commercialisé par SILAB sous la dénomination Vitanol®; un extrait peptidique de noisette tel que celui commercialisé par la société Solabia sous la dénomination Nuteline C®; l'arginine ; un extrait d'aphanizomenon flos-aquae (cyanophycée) commercialisée sous la dénomination Lanablue® par Atrium Biotechnologies, l'extrait de malt commercialisé par la société COLETICA sous la dénomination commerciale Collalift® le lycopène ; un extrait de litchi ; un extrait de moringa tel que l'Arganyl LS 9781 ® de Cognis ; un extrait de *vaccinium myrtillus* tel que ceux décrits dans la demande FR-A-2814950 ; le retinol et dérivés en particulier le palmitate de rétinol ; l'extrait de *Saccharomyces Cerivisiae* commercialisé par la société LSN sous la dénomination commerciale Cytovitin® ; un extrait peptidique de noisette tel que celui commercialisé par la société Solabia sous la dénomination Nuteline C®; l'acide 12-[acétyl-(3-trifluorométhyl-phényl)-amino]-3-méthyl-butyrylaminol acétique, autrement nommé N-[N-acétyl, N'-(3-trifluorométhyl)phényl valyl]glycine ou N-acetyl-N-[3-(trifluoromethyl)phenyl]valylglycine ou acetyl trifluoromethyl phenyl valylglycine, ou un ester de celui-ci avec un alcool en $C_1$-$C_6$. un extrait de peptides de riz tel que la Colhibin® de Pentapharm, ou un extrait de Phyllanthus emblica tel que l'Emblica® de Rona ; l'extrait d'algue brune Padina pavonica commercialisé par la société ALBAN MÜLLER sous la dénomination commerciale HSP3® ; l'extrait de *Saccharomyces cerevisiae* disponible notamment auprès de la société SILAB sous la dénomination commerciale Firmalift® ou auprès de la société LSN sous la dénomination commerciale Cytovitin® ; un extrait de *Laminaria ochroleuca* tel que la Laminaïne® de Secma ; l'essence de Mamaku de Lucas Meyer, l'extrait de lupin commercialisé par la société SILAB sous la dénomination commerciale Structurine® ; l'extrait de bourgeons de hêtre *Fagus sylvatica* commercialisé par la société GATTEFOSSE sous la dénomination commerciale Gatuline® RC.

<u>Agents stimulant la prolifération des fibroblastes ou des kératinocytes et/ou la différenciation des kératinocytes</u>

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; et les hormones végétales telles que les giberrellines et les cytokinines ; un extrait peptidique de noisette tel que celui commercialisé par la société Solabia sous la dénomination Nuteline C®.
De préférence on utilisera un agent favorisant la prolifération et/ou la différenciation des kératinocytes.
Les agents stimulant la prolifération des kératinocytes, utilisables dans la composition selon l'invention, comprennent notamment ; le phloroglucinol, l'extrait de feuille d'hydrangea macrophylla comme l'Amacha liquid E® d'Ichimaru Pharcos, un extrait de levure tel que le Stimoderm® de CLR ; l'extrait de *Larrea divaricata* tel que le Capislow® de Sederma, les mélanges d'extraits de papaye, de feuilles d'olivier et de citron tel que la Xyléine® de Vincience, l'extrait de feuille d'hydrangea macrophylla comme l'Amacha liquid E® d'Ichimaru Pharcos, le rétinol et ses esters dont le palmitate de rétinyle, les extraits de tourteaux de noix commercialisés par Gattefosse et les extraits de *solanum tuberosum* tel que le Dermolectine® commercialisé par Sederma.
Parmi les agents stimulant la différenciation des kératinocytes comprennent par exemple les minéraux tels que le calcium; un extrait peptidique de lupin tel que celui commercialisé par la société SILAB sous la dénomination commerciale Structurine®; le beta-sitosteryl sulfate de sodium tel que celui commercialisé par la société SEPORGA sous la dénomination commerciale Phytocohésine® ; et un extrait hydrosoluble de maïs tel que celui commercialisé par la société SOLABIA sous la dénomination commerciale Phytovityl® ; un extrait peptidique de *Voandzeia substerranea* tel que celui commercialisé par la société Laboratoires Sérobiologiques sous la dénomination commerciale Filladyn LS 9397® ; et les lignanes tels que le sécoisolaricirésinol, le rétinol et ses esters dont le palmitate de rétinyl.
Comme agents stimulant la prolifération et/ou la différenciation des kératinocytes, on peut citer encore les oestrogènes tel que l'oestradiol et homologues ; les cytokines.
Comme actifs stimulant la prolifération des fibroblastes ou des kératinocytes et/ou la différenciation des kératinocytes préférés, on citera des protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; un extrait peptidique de noisette tel que celui commercialisé par la société Solabia sous la dénomination Nuteline C®; l'adénosine, le phloroglucinol, un extrait de levure tel que le Stimoderm® de CLR ; un extrait peptidique de lupin tel que celui commercialisé par la société SILAB sous la dénomination commerciale Structurine®; un extrait hydrosoluble de maïs tel que celui commercialisé par la société SOLABIA sous la dénomination commerciale Phytovityl® ; un extrait peptidique de *Voandzeia substerranea* tel que celui commercialisé par la société Laboratoires Sérobiologiques sous la dénomination commerciale Filladyn LS 9397® ; le rétinol et ses esters dont le palmitate de rétinyl.

<u>Agents favorisant la maturation de l'enveloppe cornée</u>

On pourra utiliser dans les compositions de l'invention des agents qui interviennent sur la maturation de l'enveloppe cornée qui s'altère avec l'âge et induit une diminution de l'activité des transglutaminases. On peut citer par exemple l'urée et ses dérivés et en particulier l'Hydrovance® de National Starch et les autres actifs mentionnés dans la demande L'OREAL FR2877220.

Inhibiteurs de NO-synthases

L'agent ayant une action d'inhibiteur de NO synthase peut être choisi parmi les OPC (oligomères procyanidoliques) ; les extraits de végétal de l'espèce *Vitis vinifera* notamment commercialisés par la société Euromed sous la dénomination Leucocyanidines de raisins extra, ou encore par la société Indena sous la dénomination Leucoselect®, ou enfin par la société Hansen sous la dénomination Extrait de marc de raisin ; les extraits de végétal de l'espèce *Olea europaea* de préférence obtenus à partir de feuilles d'olivier et notamment commercialisés par la société VINYALS sous forme d'extrait sec, ou par la société Biologia & Technologia sous la dénomination commerciale Eurol® BT ; les extraits d'un végétal de l'espèce *Gingko biloba* de préférence un extrait aqueux sec de ce végétal vendu par la société Beaufour sous le nom commercial Ginkgo biloba extrait standard et leurs mélanges.

Agents stimulant le métabolisme énergétique des cellules

L'actif stimulant le métabolisme énergétique des cellules peut par exemple être choisi parmi la biotine, un extrait de *Saccharomyces cerevisiae* tel que le Phosphovital® de Sederma, le mélange de sels de sodium, de manganèse, de zinc et de magnésium d'acide pyrrolidone carboxylique comme le Physiogenyl® de Solabia, un mélange de gluconate de zinc, de cuivre et de magnésium tel que le Sepitonic M3® de Seppic et leurs mélanges ; et un beta-glucan issu de *Saccharomyces cerevisiae,* tel que celui commercialisé par la société Mibelle AG Biochemistry.

L'invention porte également sur un procédé de traitement cosmétique de la peau destiné à diminuer ou prévenir les signes du vieillissement de la peau ou de ses phanères (cheveux, cils, ongles, ...), comprenant au moins une étape consistant à appliquer sur la peau au moins une composition telle que définie précédemment.

Le procédé selon l'invention comprend plus spécifiquement au moins une étape consistant à appliquer sur la peau de personnes présentant une peau présentant au moins l'un des signes de vieillissement cutané rappelés précédemment au moins une composition telle que définie précédemment.

Plus particulièrement, il comprend au moins une étape consistant à appliquer sur la peau de personnes présentant une peau ou une zone de peau vieillie, ridée, ou molle et/ou flasque ou sur des zones du corps présentant une perte d'élasticité et/ou de fermeté et/ou de tonicité au moins une composition telle que définie précédemment.

La composition selon l'invention peut être appliquée sur la partie de la peau ou des phanères à traiter, en particulier sur le visage, le corps, le cou, les mains, les cheveux ou le cuir chevelu, de préférence de façon quotidienne ou pluriquotidienne. L'application pourra être par exemple renouvelée tous les jours pendant une période variable selon les effets souhaités, généralement de 3 à 6 semaines, mais pourra être prolongée ou poursuivie en continu.

Selon un aspect particulier, l'invention concerne également un ensemble cosmétique comprenant : i) un récipient délimitant au moins un compartiment, ledit récipient étant fermé par un élément de fermeture ; et ii) une composition telle que décrite précédemment et disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier. L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur. L'applicateur peut être sous forme d'un pinceau, tel que décrit par exemple dans le brevet FR 2 722 380. Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage.

Alternativement, le couplage entre l'élément de fermeture et le récipient se fait autrement que par vissage, notamment via un mécanisme à baïonnette, par encliquetage, serrage, soudage, collage, ou par attraction magnétique. Par " encliquetage " on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique, comme le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient.

Le contenu des brevets ou demandes de brevets cités précédemment sont incorporés par référence dans la présente demande.

Selon un autre mode particulier, l'invention concerne un ensemble, en particulier cosmétique, comprenant :

- une composition A contenant au moins un filtre solaire,
- une composition B, conditionnée de manière séparée de la composition A, comprenant au moins un monosaccharide choisi parmi le mannose, le rhamnose et leur mélange.

L'invention concerne enfin un procédé de traitement, cosmétique ou thérapeutique, comprenant au moins une étape d'application sur la peau et/ou ses phanères de la composition A et au moins une étape d'administration par voie orale, topique ou par injection cutanée de la composition B.

Les compositions A et/ou B sont des compositions telles que décrites précédemment. La composition A selon l'invention est adaptée à une administration topique sur la peau ou ses phanères. La composition B selon l'invention est adaptée à une administration topique sur la peau ou ses phanères, une administration orale ou une injection cutanée, en particulier sous la forme d'une solution stérile. De manière préférée, les compositions orales selon l'invention se présentent sous la forme d'une gélule, d'un comprimé, ou de pilules.

De manière générale, les compositions A et B peuvent être administrées de manière simultanée, consécutive ou décalée dans le temps. Selon une alternative, on applique sur la peau ou ses phanères en premier lieu la composition A et en second lieu on administre par voie topique sur la peau ou ses phanères, par voie orale ou par injection cutanée en second lieu la composition B. Inversement, on peut administrer en premier lieu la composition B et en second lieu appliquer la composition A.

Les compositions A et B peuvent être conditionnées séparément à l'intérieur de deux compartiments, formés soit par deux récipients distincts, soit à l'intérieur d'un dispositif unitaire. Par " dispositif unitaire " on entend un dispositif par lequel les deux compartiments sont solidaires l'un de l'autre. Un tel dispositif peut être obtenu par un procédé de moulage en une seule pièce des deux compartiments, notamment en un matériau thermoplastique. Il peut également résulter de toute forme d'assemblage, notamment par collage, soudage, ou autre encliquetage.

Selon un premier mode de réalisation, les deux récipients sont indépendants l'un de l'autre. De tels récipients peuvent se présenter sous diverses formes. Il peut s'agir notamment de tubes, de flacons ou de bidons.

L'un et/ou l'autre des récipients peuvent être surmontés d'une pompe à actionnement manuel surmontée d'un bouton poussoir pour l'actionnement de la pompe et la distribution de la composition via au moins un orifice de distribution.

Alternativement, l'un et/ou l'autre des récipients sont pressurisés, notamment au moyen d'un agent propulseur, en particulier un gaz propulseur. Dans ce cas, le (ou les) récipient(s) est (sont) équipé(s) d'une valve surmontée par un bouton poussoir équipée d'une buse ou de tout autre moyen de diffusion pour la distribution du produit.

Le propulseur peut être en mélange avec la composition à distribuer ou séparé, notamment via un piston apte à coulisser à l'intérieur du récipient, ou via les parois souples d'une poche à l'intérieur de laquelle est disposée la composition.

Les récipients peuvent être constitués de matériaux divers : plastique, verre, ou métal.

Alternativement encore, les deux compartiments sont formés de deux compartiments concentriques formés à l'intérieur d'un tube, et sont surmontés d'une pompe sans reprise d'air équipée d'un bouton poussoir à un ou deux orifices de distribution. A l'intérieur du tube est prévu un piston qui remonte en direction de la pompe au fur et à mesure que les compositions sont prélevées à l'intérieur des récipients. De tels modes de distribution sont utilisés notamment pour la distribution de pâtes dentifrices.

Selon une alternative, la composition B dudit ensemble cosmétique selon l'invention peut être administrée par voie injectable en association ou non avec des produits de comblement. En effet, l'une des solutions retenues pour lutter contre les rides et/ou la perte de volume des tissus mous est l'utilisation de produits de comblement (ou "filler"). Ce comblement peut être réalisé par l'utilisation de produits non résorbables, tels que des gels de polyacrylamide ou des particules de polyméthylméthacrylate (PMMA). Néanmoins, ces composés peuvent entrainer des réactions d'intolérance du type inflammation ou hypersensibilité.

On privilégie l'utilisation de composants résorbables, tels que les protéines, les graisses, le collagène ou l'acide hyaluronique. Mais ces composés sont dégradés assez rapidement dans l'organisme, ce qui réduit leur efficacité. Pour y remédier il faut donc procéder à une réticulation plus ou moins poussée de ces composants. A ce jour, l'acide hyaluronique utilisé dans des formes pharmaceutiques ou des dispositifs médicaux se présente sous la forme d'un gel de hyaluronate de sodium. Le monosaccharide selon l'invention ou les compositions le contenant pourront également être appliqués par mésothérapie. La mésothérapie est une technique de traitement par injection intraépidermique et/ou intradermique et/ou sous-cutanée de produit(s) actif(s), comme par exemple des micro-nutriments, des vitamines, et/ou de l'acide hyaluronique. Les compositions sont administrées selon cette technique par injection sous forme de multiples gouttelettes de faible taille au niveau de l'épiderme, de la jonction dermo-épidermique et/ou du derme afin, notamment, de réaliser un nappage sous-cutané. La technique de mésothérapie est notamment décrite dans l'ouvrage « Traité de mésothérapie » de Jacques LE COZ, édition Masson, 2004. La mésothérapie faite sur le visage est également appelée mésolift, ou également sous le terme anglosaxon de « mesoglow ».

Ainsi, un objet particulier de la présente invention peut être un ensemble comprenant une composition A et une composition B, tel que décrit ci-avant, dans lequel la composition B est sous forme de dispositif, en particulier un dispositif médical, comprenant une quantité efficace d'au moins un monosaccharide tel que défini précédemment. Ce dispositif peut être adapté à une injection intraépidermique et/ou intradermique et/ou sous-cutanée. Le monosaccharide tel que défini ci-dessus est mis en solution dans un milieu stérile. Ledit dispositif peut comprendre au moins un autre composé, comme au moins un produit résorbable ou non, tel que ceux mentionnés ci-dessus, éventuellement réticulé.

Ledit dispositif peut être par exemple une seringue avec une aiguille ou encore un dispositif injecteur sans aiguille, tel que ceux utilisés dans la technique de soin connue sous le nom de mésothérapie. Un kit comprenant un dispositif peut être également envisagé, ledit kit comprenant un dispositif, en particulier une seringue ou un dispositif injecteur, au moins le monosaccharide et au moins une composition A telle que définie ci-avant, ladite composition A étant destinée à être appliquée de manière topique sur la peau ou ses phanères. Ledit kit peut comprendre également une aiguille. Ledit dispositif peut se trouver prêt à l'emploi, c'est à dire pré-rempli, ou doit être rempli lors de l'utilisation. Dans ce dernier cas, une composition ou un autre dispositif (comme une ampoule) comprend ledit monosaccharide, éventuellement en association avec au moins un autre composé actif, comme au moins un produit résorbable ou non, tel que les produits de comblement mentionnés ci-dessus, éventuellement réticulé.

L'injection du monosaccharide selon l'invention peut être réalisé simultanément à, ou avant ou après, l'application sur la peau ou ses phanères d'une autre composition cosmétique ou pharmaceutique, de préférence dermatologique, comprenant, dans un support physiologiquement acceptable, au moins un autre actif, tel que cité ci-dessus.

## Légendes des Figures

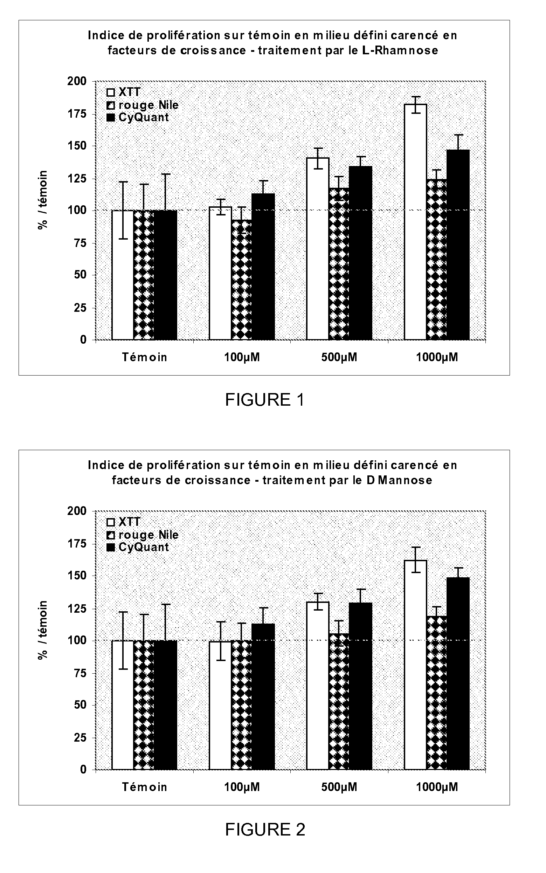

**Figure 1 :** Diagramme schématisant les résultats obtenus pour la prolifération des kératinocytes, en présence d'un témoin, en présence de différents marqueurs, en milieu carencé en facteurs de croissance, et avec ajout de différentes concentrations en L-Rhamnose reportées en abscisse. Les valeurs reportées en ordonnée correspondent aux pourcentages de cellules marquées mesurés par rapport au témoin.

**Figure 2 :** Diagramme schématisant les résultats obtenus pour la prolifération des kératinocytes, en présence d'un témoin, en présence de différents marqueurs, en milieu carencé en facteurs de croissance, et avec ajout de différentes concentrations en D-Mannose reportées en abscisse. Les valeurs reportées en ordonnée correspondent aux pourcentages de cellules marquées mesurés par rapport au témoin.

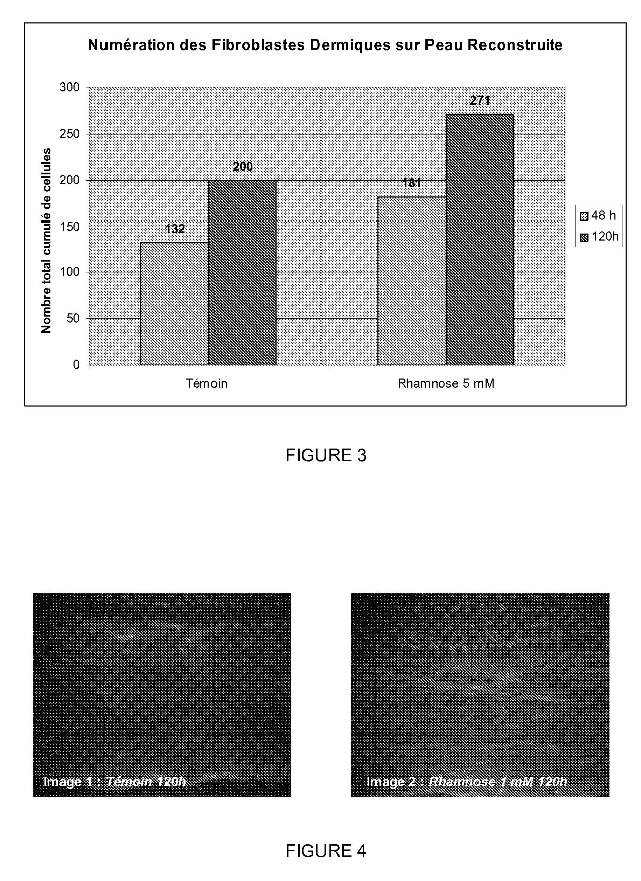

**Figure 3 :** Diagramme représentant le nombre de fibroblastes mesurés entre une peau reconstruite complète témoin non traité, à gauche, et une peau reconstruite complète traitée par 5 mM de rhamnose, à droite. Les fibroblastes sont comptés à différents stades du traitement. Ainsi, pour chaque type de peau la colonne de gauche correspond à la numérotation effectuée à 48 h et la colonne de droite correspond à la numérotation effectuée à 120 h de traitement.

**Figure 4 :** Photographies de coupe à congélation de peau reconstruite de 7 $\mu$m d'épaisseur. Le niveau de fluorescence se matérialise par les tâches blanche du cliché en noir et blanc, il est proportionnel à la quantité de procollagène de type 1. A gauche figure la peau témoin et à droite la peau traitée par 1 mM de rhamnose.

L'invention est illustrée plus en détail dans les exemples suivants qui sont présentés à titre illustratif et non limitatif du domaine de l'invention.

## Exemples

### Exemple 1 : **Prolifération des kératinocytes**

**Protocole**

Les kératinocytes (lignée HaCat) sont cultivés dans deux conditions : milieu de culture défini complet (condition standard) et milieu de culture carencé en facteurs de croissance. Ce milieu carencé entraîne un retard maitrisé de la prolifération cellulaire. Dans ces conditions, il est alors possible de mesurer les effets de composés, capables de compenser la carence en facteurs de croissance du milieu de culture et donc de relancer la multiplication cellulaire et /ou de stimuler leur métabolisme.

La prolifération kératinocytaire est mesurée au moyen de trois marqueurs sur la même population cellulaire : le taux d'ADN qui est proportionnel au nombre de cellules (sonde Cyquant), le taux de lipides polaires constitutifs de membranes cellulaires (sonde Rouge Nil) et la respiration mitochondriale, qui réflète le métabolisme cellulaire général (sonde XTT).

**Résultats**

Les résultats sont donnés dans les figures 1 et 2.

Les deux monosaccharides Rhamnose et Mannose démontrent leur capacité à activer la prolifération des kératinocytes, lorsque ceux-ci sont cultivés en milieu appauvri en facteurs de croissance, condition de culture qui retarde significativement leur croissance cellulaire.

Cette activation de la prolifération cellulaire par les deux composés se manifeste par un nombre de cellules plus important par rapport au témoin non traité.

Ce nombre augmenté de cellules se matérialise par un taux d'ADN (Cyquant), un taux de lipides polaires (signal Rouge Nil) et une respiration mitochondriale (signal XTT) significativement augmentés lorsque les monosaccharides sont évalués à 1 mM. A 500 $\mu$M, les deux molécules présentent déjà une efficacité.

Les deux monosaccharides mannose et rhamnose exercent donc une influence sur la prolifération des kératinocytes. Ils activent la prolifération des kératinocytes cultivés en milieu appauvri en facteur de croissance, ce qui se manifeste par un nombre de cellules plus important par rapport au témoin non traité.

Le rhamnose et le mannose présentent donc une efficacité anti âge intéressante en venant booster le renouvellement épidermique et lutter contrer l'atrophie épidermique liée au vieillissement.

**Exemple 2** : **Prolifération des fibroblastes**

**Protocole**

Le rhamnose a été étudié sur un modèle de peau reconstruite complète afin de mesurer son efficacité anti âge au niveau du compartiment dermique.

Brièvement, le modèle de peau reconstruite utilisé est celui décrit par Bell et al, (Bell E. et al, The reconstitution of living skin, J Invest Dermatol, 1983, Jul ;81*)* : il comporte un équivalent dermique sur lequel est reconstruit un épiderme pluristratifié ; l'équivalent dermique est fabriqué à partir de collagène acido soluble, de milieu de culture contenant du sérum et de fibroblastes humains normaux adultes. Après 5 jours de rétraction, cet équivalent est ensemencé avec des kératinocytes puis cultivé durant 6 jours en immersion et 7 jours à émersion afin d'obtenir un épiderme pluristratifié et différencié présentant une couche cornée.

La peau reconstruite est traitée par du rhamnose à 5 mM pendant 2 jours et 5 jours dans le milieu de culture ; à l'issue du traitement, les peaux reconstruites sont incluses en Tissue Tek en vue de coupes à congélation de 7 $\mu$m d'épaisseur au cryostat. Les coupes réalisées sont ensuite marquées à l'iodure de propidium pour marquer l'ADN des noyaux des fibroblastes en vue de leur numération. 3 coupes à congélation sont réalisées aléatoirement sur chaque peau reconstruite ; sur chaque coupe, 2 champs microscopiques (objectif X25) sont analysés en microscopie à fluorescence et photographiés. La numération des fibroblastes dermiques est donc réalisée pour chaque peau reconstruite sur 6 images au total représentant les 6 champs microscopiques considérés. Le nombre de fibroblastes dermiques est comparé entre la peau témoin et celle traitée par le rhamnose aux deux temps de la cinétique.

**Résultats**

Les résultats sont donnés à la figure 3.

Il a été constaté que le rhamnose induit la stimulation de la croissance des fibroblastes dermiques de la peau reconstruite dès 48 heures de traitement, stimulation confirmée à 120 h de traitement, avec entre 30 à 35% de cellules en plus (voir figure 3). A noter que cette stimulation s'accompagne d'une stimulation de la synthèse de procollagène 1 à 5 mM, ainsi qu'à 1 mM, ce qui peut également résulter du nombre accru des fibroblastes responsable de la sécrétion de cette protéine majeure de la matrice extracellulaire.

Ces deux effets complètent l'activité anti-âge du rhamnose déjà mesurée sur le compartiment épidermique, en venant stimuler la prolifération et le métabolisme du fibroblaste, cellule majeure du compartiment dermique.

**Exemple 3 : Synthèse de Procollagène 1**

Il a été procédé également sur d'autres séries de coupes à congélation à la détection classique par immunofluorescence indirecte du procollagène de type I au niveau du derme de la peau reconstruite (Anticorps anti procoll 1 *(MAB 1912 Millipore)* + conjugué couplé à FITC (*112-095-068 Jackson Immunoresearch*)). Afin de bien se repérer au sein de l'architecture cutanée lors de l'examen microscopique des coupes, les noyaux cellulaires des kératinocytes et des fibroblastes sont localisés grâce à leur marquage à l'iodure de propidium, comme décrit plus haut. 3 coupes à congélation sont réalisées aléatoirement sur chaque peau reconstruite et sur chaque coupe, 2 champs microscopiques (objectif X25) sont analysés en microscopie à fluorescence et photographiés. Les niveaux de fluorescence proportionnels à la quantité de procollagène de type I sont comparés entre la peau témoin et la peau traitée par le rhamnose.

Sur l'image 1, figure 4, correspondant à une coupe de la peau reconstruite témoin à 120h de culture, la présence du procollagène de type 1 synthétisé par les fibroblastes dermiques, se matérialise par la fluorescence verte située dans la partie inférieure de l'image. On devine sur la partie supérieure de l'image, la partie basale de l'épiderme, tissu très cellulaire, visualisable par les nombreux noyaux des kératinocytes. On visualise également dans le derme ; tissu beaucoup moins cellulaire, la distribution aléatoire des fibroblastes au sein de la matrice extracellulaire dermique. Sur l'image 2, figure 4, correspondant par exemple, à une coupe de la peau reconstruite traitée par le Rhamnose à 1mM pendant 120 heures, on constate une nette augmentation de la fluorescence verte comparativement à celle observée pour la peau témoin (image 1) ainsi qu'une distribution du signal fluorescent matérialisant bien l'aspect fibrillaire du procollagène de type I néosynthétisé. Cette augmentation de la fluorescence générale indique que le traitement par le rhamnose a fortement stimulé la synthèse du procollagène de type I par les fibroblastes.

Ces résultats montrent bien la capacité du rhamnose à stimuler le métabolisme du fibroblaste, métabolisme qui au cours du vieillissement, se déséquilibre plus vers la dégradation de la matrice extracellulaire que vers son renouvellement.

Le rhamnose en stimulant à la fois le métabolisme et la croissance des fibroblastes dermiques démontre bien son efficacité anti-âge sur le derme, efficacité complémentaire de celle mesurée vis-à-vis du compartiment épidermique.

**Exemple 4** : **Association Rhamnose et d'un filtre solaire (Mexoryl SX) : Mise en évidence de la complémentarité d'action anti âge du rhamnose et d'un filtre solaire (Mexoryl SX)**

♦ *Protocole : protection vis-à-vis de la mortalité cellulaire UV induite.*

L'association de filtres UV avec un monosaccharide est évaluée sur kératinocytes en culture vis-à-vis de la mortalité cellulaire UV induite.

Brièvement, les kératinocytes HaCaT sont cultivés à confluence en plaque 12 puits en milieu de base DMEM/ Glutamax 1, (Gibco cat n°21885-025) + SVF, (Gibco cat n°10270-098) + Fungizone Ampb B (Gibco cat n°15290081) + Plasmocin (TebuBio), à 37°C / 5% $CO_2$. Les cellules sont ensuite incubées en présence du monosaccharide d'intérêt à 1 et/ou 5 mM pendant 24 heures précédemment à l'exposition UV. Les cellules peuvent également être traitées par la molécule en post traitement, soit ultérieurement à l'exposition UV.

Une formule de protection solaire de SPF égal à 6.5, contenant le système filtrant composé de l'association de 3% d'Octocrylène, 1 % de Mexoryl SX et 2.4% Uvinul A+, est appliquée uniformément à raison de 0,6 mg/cm$^2$ sur une plaque de PMMA, qui est ensuite déposée au dessus de la plaque de culture 12 puits contenant les kératinocytes.

Les kératinocytes traités par le rhamnose, en présence ou pas des filtres UV appliqués sur plaque de quartz, sont ensuite soumis à l'exposition UV au moyen d'un simulateur solaire ORION de marque Oriel muni du filtre WG335 délivrant l'ensemble du spectre des UVA et du visible. Les cellules sont exposées à deux doses d'UVA comme par exemple, 10 et 20 J/cm$^2$. Des cellules témoins sont conservées à l'obscurité.

Suite à l'exposition UV, la viabilité cellulaire est mesurée par le test au Rouge Neutre. Le rouge neutre est un colorant d'inclusion spécifique des lysosomes. L'accumulation de ce colorant dépend de l'intégrité membranaire des cellules. Il est ainsi possible de distinguer les cellules vivantes (incorporation du rouge neutre) des cellules mortes ou endommagées, et de les quantifier. Les cellules sont alors incubées en présence de rouge neutre pendant 1 heure à 37°C et 5% de $CO_2$. Le colorant est ensuite extrait des cellules par addition d'une solution d'éthanol 50% et de $NaH_2PO_4$ 0,05M. La lecture de l'absorbance est réalisée au spectrophotomètre à 540 nm.

♦ *Protocole : évaluation de l'association Rhamnose + Filtres solaires vis-à-vis des UVA sur Peau Reconstruite (mortalité des fibroblastes du derme superficiel)*

La peau reconstruite utilisée est produite par la société EPISKIN SNC (Lyon). Elle est réalisée selon le protocole décrit dans Asselineau et al. (Models in Dermato. Editions Loire and Maibach, 1987, Vol III, 1-7), avec certaines modifications qui sont :

- l'utilisation de fibroblastes de derme humains normaux adultes à raison de $10^6$ cellules par derme équivalent ;
- l'ensemencement des kératinocytes se fait à raison de 50000 cellules par anneau de 1.5 cm de diamètre. Les kératinocytes utilisés proviennent d'un même donneur et sont en passage 1 lors de l'ensemencement des équivalents de derme
- la durée de la phase d'immersion est de 7 jours ;
- la durée de la phase d'émersion est de 7 jours.

La Peau Reconstruite est traitée pendant 2 jours par le rhamnose à 5 mM dans le milieu de culture. Une formule de protection solaire de SPF égal à 6.5, contenant le système filtrant composé de l'association de 3% d'Octocrylène, 1 % de Mexoryl SX et 2.4% Uvinul A+, est appliquée uniformément à raison de 2 mg/cm$^2$ à la surface de la Peau Reconstruite ou sur une plaque de PMMA déposée au dessus de la Peau Reconstruite. Plusieurs séries de peau sont ainsi préparées : peau Témoin, peau traitée par le rhamnose, peau traitée par la formule solaire, peau traitée par le rhamnose et la formule solaire.

Les différentes séries de peau reconstruite sont exposées aux UVA au moyen d'un simulateur solaire ORION de marque Oriel muni du filtre WG335 délivrant l'ensemble du spectre des UVA et du visible. Les Peaux Reconstruites sont exposées aux UVA à la dose de 20 J/cm$^2$. Des peaux témoins sont conservées à l'obscurité.

Postérieurement à l'exposition UV, les peaux reconstruites sont retraitées par le rhamnose au minimum pendant 72 heures, voire jusqu'à 10 jours post expo UV, avec un changement du milieu de culture tous les 2 jours.

A l'issue du traitement, les différentes séries de peaux reconstruites sont préparées en vue de réaliser des coupes à congélation de 7μM d'épaisseur, 3 à 4 coupes sont aléatoirement réalisées par échantillon de peau ; après séchage des coupes pdt 1 heures à TA et fixation à l'acétone à - 20 °C pendant 10 minutes, on procède à l'immunomarquage de la vimentine, protéine spécifique du cytosquelette des fibroblastes, afin de localiser et dénombrer les fibroblastes dermiques (anticorps de souris anti vimentine, réf MON3005-1, dilution au 1/300) + anticorps de rat anti souris FITC (ref JACKSON 112095068, dilution au 1/200). Les coupes de peaux sont observées au microscope à fluorescence Zeiss Axiovert M200.

**Résultats**

*Protection vis-à-vis de la mortalité cellulaire UV induite.*

L'association Rhamnose/filtres solaires protège les kératinocytes vis-à-vis des effets délétères des UV, notamment de la mortalité induite par le rayonnement UV.

*Evaluation de l'association Rhamnose + Filtres solaires vis-à-vis des UVA sur Peau Reconstruite (mortalité des fibroblastes du derme superficiel)*

Les UVA entrainent la disparition des Fibroblastes du derme superficiel ; par contre, les cellules persistent dans la partie inférieure du compartiment dermique comme en témoigne l'immunomarquage de la vimentine, protéine du cytosquelette du fibroblaste.

Lorsque la formule solaire est appliquée en surface, on constate une protection partielle des fibroblastes vis-à-vis de la mortalité induite par les UVA.

Par contre, lorsque la formule solaire est associée au rhamnose, on mesure un niveau de protection supérieur. On mesure bien les effets complémentaires des filtres solaires et du monosaccharide pour protéger le derme des effets délétères des UV.

**Exemple 5 : exemple de réalisation d'une composition cosmétique selon l'invention**

| Crèmes ANTI Age : émulsion huile dans eau | |
|---|---|
| Ammonium Polyacryldimethyltauramide (Hostacerin AMPS de Clariant) | 1,00% |
| Isononyl isononanoate | 3% |
| Isopropyl Lauroyl sarcosinate | 7% |
| Cyclohexasiloxane | 5,0% |
| Glycerin | 1,70% |

(suite)

| Crèmes ANTI Age : émulsion huile dans eau | |
|---|---|
| Stearyl alcohol | 0,30% |
| Glyceryl stearate / PEG-100 stearate | 0,70% |
| Dimyristyl tartrate / cetearyl alcohol / C12-15 pareth-7 / PPG-25 laureth-25 | 0,50% |
| Octocrylen | 5% |
| Butylmethoxydibenzoyl methane | 1,5% |
| Gomme de Xanthan | 0,20 % |
| rhamnose | 5% |
| Terephthalylidene dicamphor sulfonic acid | 1,5% |
| Conservateurs | 0,3% |
| Eau | qsp 100 |

Lorsque cette composition est appliquée bi-quotidiennement pendant 6 mois, une amélioration globale de l'âge apparent du visage, via en particulier une réduction de l'apparence des rides et une amélioration de l'éclat du teint.

**Exemple 6 : exemple de réalisation d'une composition cosmétique selon l'invention**

| Crème Anti Age : émulsion huile dans eau | |
|---|---|
| Ammonium Polyacryldimethyltauramide (Hostacerin AMPS de Clariant) | 1,00% |
| Cyclohexasiloxane | 5,0% |
| Isononyl isononanoate | 3% |
| Isopropyl Lauroyl sarcosinate | 7% |
| Stearyl alcohol | 0,30% |
| Glyceryl stearate / PEG-100 stearate | 0,70% |
| Dimyristyl tartrate / cetearyl alcohol / C12-15 pareth-7 / PPG-25 laureth-25 | 0,50% |
| Octocrylen | 7% |
| Butylmethoxydibenzoyl methane | 3% |
| Ethyl hexyl salicylate | 5% |
| Gomme de Xanthan | 0,20 % |
| mannose | 5% |
| Conservateurs | 0,50% |
| Eau | qsp 100 |

**Exemple 7 : exemple de réalisation d'une composition cosmétique selon l'invention**

| Crème Anti Age : émulsion huile dans eau | |
|---|---|
| Ammonium Polyacryldimethyltauramide (Hostacerin AMPS de Clariant) | 1,00% |
| Cyclohexasiloxane | 5,0% |

(suite)

| Crème Anti Age : émulsion huile dans eau | |
|---|---|
| Isononyl isononanoate | 3% |
| Isopropyl Lauroyl sarcosinate | 7% |
| Stearyl alcohol | 0,30% |
| Glyceryl stearate / PEG-100 stearate | 0,70% |
| Dimyristyl tartrate / cetearyl alcohol / C12-15 pareth-7 / PPG-25 laureth-25 | 0,50% |
| Octocrylen | 7% |
| Butylmethoxydibenzoyl methane | 3% |
| Ethyl hexyl salicylate | 3% |
| Dioxyde de titane | 3% |
| Gomme de Xanthan | 0,20 % |
| Mannose | 2,5% |
| Rhamnose | 2,5% |
| Conservateurs | 0,50% |
| Eau | qsp 100 |

**Exemple 8** : **exemple de réalisation d'une composition cosmétique selon l'invention**

Crème de jour anti-âge pour le visage

Phase A1:-
- Distéarate de sucrose commercialisé par la Société STEARINERIE DUBOIS                    1,75 %
- Stéarate de sorbitane oxyéthyléné à 4 moles d'oxyde d'éthylènecommercialisé par la Société ICI
sous le nom "TWEEN 61"                    1,15 %
- Acide stéarique                    0,75 %
- Stearyl heptanoate                    4,00 %
- Vaseline codex                    1,50 %
- Isopropyl lauroyl sarcosinate                    7,00 %
- Huile de jojoba                    3,00 %
- Huile de silicone volatile                    2,70 %
- Acétate de vitamine E                    1,00 %
- Glycérides de Vitamine F                    3,00 %
- Ethyl hexyl salicylate                    5%
- Butylmethoxydibenzoyl methane                    3%
- Octocrylen                    5%
Phase A2 :
Gomme de silicone commercialisée par DOW CORNING sous le nom "Q2-1403 Fluid"                    3,00 %
- Propylparaben                    0,2 %
- Parfum                    0,3 %
Phase B :
- Glycérine                    3,00 %
- Hydroxyproline                    1,00 %
- D-panthenol                    1,00 %
- Triéthanolamine                    0,35 %

(suite)

Phase B :
| | | |
|---|---|---|
| - Rhamnose | | 3,00% |
| - Méthylparaben | | 0,3 % |
| - Eau déminéralisée | q.s.p. | 100 % |

Phase C :
| | |
|---|---|
| - Ammonium Polyacryldimethyltauramide (Hostacerin AMPS de Clariant) | 1% |

**Revendications**

1. Utilisation cosmétique d'une composition comprenant, dans un milieu physiologiquement acceptable, l'association d'au moins un monosaccharide choisi parmi le mannose, le rhamnose et leur mélange, et d'au moins un filtre solaire, pour diminuer et/ou prévenir les signes du vieillissement de la peau ou de ses phanères.

2. Utilisation cosmétique selon la revendication 1, pour améliorer l'éclat du teint, pour diminuer et/ou prévenir les caractéristiques des rides et/ou ridules, pour diminuer et/ou prévenir les taches pigmentaires, pour améliorer et/ou diminuer le micro-relief de la peau, pour lisser la peau, pour améliorer les propriétés mécaniques de la peau et/ou pour favoriser la réparation de la peau.

3. Utilisation selon l'une des revendications 1 et 2, pour améliorer la densité de la peau et/ou sa fermeté.

4. Utilisation selon l'une quelconque des revendications 1-3, pour traiter, de manière préventive ou curative, les rides et/ou ridules, la peau flétrie, le manque d'élasticité et/ou de tonus de la peau, l'amincissement du derme, la dégradation des fibres de collagène, la peau molle et/ou la peau amincie.

5. Utilisation selon l'une quelconque des revendications 1-4, pour augmenter la synthèse des collagènes, de préférence le pro-collagène I.

6. Composition cosmétique comprenant, dans un milieu physiologiquement acceptable, l'association d'au moins un monosaccharide choisi parmi le mannose, le rhamnose et leur mélange, et d'au moins un filtre solaire, ladite composition ne comprenant pas l'association de xylitol et de mannitol, dans laquelle le filtre solaire est un filtre organique choisi parmi les anthranilates ; les dérivés salicyliques ; les dérivés de la benzophénone ; les dérivés de diphénylacrylate ; les dérivés de triazine ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'$\alpha$-alkylstyrène ; les 4,4-diarylbutadiènes ; les dérivés de mérocyanines et leurs mélanges.

7. Composition cosmétique comprenant, dans un milieu physiologiquement acceptable, l'association d'au moins un monosaccharide choisi parmi le mannose, le rhamnose et leur mélange, et d'au moins un filtre solaire choisi parmi :

   Ethylhexyl Methoxycinnamate,
   Ethylhexyl Salicylate,
   Homosalate,
   Octocrylene,
   Phenylbenzimidazole Sulfonic Acid,
   Benzophenone-3,
   2-(4-diethylamino-2-hydroxybenzoyl)-benzoate de n-hexyle.
   Terephthalylidene Dicamphor Sulfonic Acid,
   Methylène bis-Benzotriazolyl Tetramethylbutylphénol,
   Bis-Ethylhexyloxyphenol Methoxyphenyl Triazine,
   Diethylhexyl Butamido Triazone,
   la 2,4,6-tris(4'-amino benzalmalonate de dinéopentyle)-s-triazine,
   la 2,4,6-tris-(4'-amino benzalmalonate de diisobutyle)-s-triazine,
   la 2,4-bis-(4'-aminobenzoate de n-butyle)-6-(aminopropyltrisiloxane)-s-triazine,

la 2,4-bis-(4'-aminobenzalmalonate de dinéopentyle)-6-(4'-aminobenzoate de n-butyle)-s-triazine,
Drometrizole Trisiloxane,
Octyl-5-N,N-diethylamino-2-phenysulfonyl-2,4-pentadienoate,

et leurs mélanges.

**8.** Composition selon l'une quelconque des revendications 6-7, dans laquelle les filtres minéraux sont des pigments d'oxydes métalliques enrobés ou non, dont la taille moyenne des particules primaires est préférentiellement comprise entre 5 nm et 100 nm (de préférence entre 10 nm et 50 nm).

**9.** Composition selon l'une quelconque des revendications 6-8, dans laquelle la quantité dudit filtre solaire est comprise entre 0,01 % et 20 % en poids par rapport au poids total de la composition, et en particulier entre 0,1% et 10 % en poids par rapport au poids total de la composition.

**10.** Composition selon l'une quelconque des revendications 6-8, dans laquelle la quantité dudit monosaccharide est comprise entre 0,3 % et 10 % et la quantité dudit filtre solaire est comprise entre 0,1 % et 20 % en poids par rapport au poids total de la composition.

**11.** Ensemble comprenant :

- une composition A contenant au moins un filtre solaire,
- une composition B, conditionnée de manière séparée de la composition A,

comprenant au moins un monosaccharide choisi parmi le mannose, le rhamnose et leur mélange.

FIGURE 1

FIGURE 2

## Numération des Fibroblastes Dermiques sur Peau Reconstruite

FIGURE 3

Image 1 : Témoin 120h

Image 2 : Rhamnose 1 mM 120h

FIGURE 4

Europäisches
Patentamt
European
Patent Office
Office européen
des brevets

**RAPPORT DE RECHERCHE EUROPEENNE**

Numéro de la demande

EP 09 18 1021

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | FR 2 756 735 A (THOREL JEAN NOEL [FR]) 12 juin 1998 (1998-06-12) * exemple 6 * ----- | 6 | INV. A61K8/60 A61Q19/08 A61K8/35 |
| X | US 2007/025933 A1 (GUERRERO GOMEZ-PAMO ANTONIO [ES] ET AL) 1 février 2007 (2007-02-01) * exemple 1; tableaux 1,2 * ----- | 13 | A61K8/37 A61K8/40 A61K8/46 |
| Y | FR 2 876 283 A (IND E COM DE COSMETICOS NATURA [BR]) 14 avril 2006 (2006-04-14) * revendications 1,11 * ----- | 1,6 | |
| Y | FR 2 739 556 A (OREAL [FR]) 11 avril 1997 (1997-04-11) * page 2, ligne 28-33 * * page 5, ligne 19-22; revendication 2 * ----- | 1,6 | |
| Y | FR 2 863 166 A (SILAB SA [FR]) 10 juin 2005 (2005-06-10) * page 4, ligne 9-13; revendications 1,3,5 * ----- | 1,6 | DOMAINES TECHNIQUES RECHERCHES (IPC) |
| A,D | FR 2 863 887 A (THOREL JEAN NOEL [FR]) 24 juin 2005 (2005-06-24) * exemples 3,5 * ----- | 6 | A61K A61Q |

Le présent rapport a été établi pour toutes les revendications

| Lieu de la recherche | Date d'achèvement de la recherche | Examinateur |
|---|---|---|
| Munich | 24 mars 2010 | Yon, Jean-Michel |

CATEGORIE DES DOCUMENTS CITES

X : particulièrement pertinent à lui seul
Y : particulièrement pertinent en combinaison avec un
  autre document de la même catégorie
A : arrière-plan technologique
O : divulgation non-écrite
P : document intercalaire

T : théorie ou principe à la base de l'invention
E : document de brevet antérieur, mais publié à la
  date de dépôt ou après cette date
D : cité dans la demande
L : cité pour d'autres raisons

 
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 09 18 1021

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

24-03-2010

| Document brevet cité au rapport de recherche | | Date de publication | Membre(s) de la famille de brevet(s) | | Date de publication |
|---|---|---|---|---|---|
| FR 2756735 | A | 12-06-1998 | AUCUN | | |
| US 2007025933 | A1 | 01-02-2007 | EP | 1762219 A1 | 14-03-2007 |
| | | | WO | 2005094773 A1 | 13-10-2005 |
| FR 2876283 | A | 14-04-2006 | WO | 2006037813 A1 | 13-04-2006 |
| FR 2739556 | A | 11-04-1997 | AT | 238032 T | 15-05-2003 |
| | | | CA | 2231553 A1 | 10-04-1997 |
| | | | DE | 69627680 D1 | 28-05-2003 |
| | | | DE | 69627680 T2 | 18-12-2003 |
| | | | EP | 0853472 A1 | 22-07-1998 |
| | | | ES | 2196178 T3 | 16-12-2003 |
| | | | WO | 9712597 A1 | 10-04-1997 |
| | | | JP | 3746298 B2 | 15-02-2006 |
| | | | JP | 11501940 T | 16-02-1999 |
| | | | US | 6391863 B1 | 21-05-2002 |
| FR 2863166 | A | 10-06-2005 | AUCUN | | |
| FR 2863887 | A | 24-06-2005 | CA | 2550671 A1 | 14-07-2005 |
| | | | CN | 1913863 A | 14-02-2007 |
| | | | EP | 1753393 A2 | 21-02-2007 |
| | | | WO | 2005063194 A2 | 14-07-2005 |
| | | | JP | 2007515462 T | 14-06-2007 |
| | | | KR | 20070000442 A | 02-01-2007 |
| | | | US | 2007128128 A1 | 07-06-2007 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2007128939 A
- FR 2900572
- US 20070025933 A
- WO 2005063194 A
- US 5624663 A
- EP 669323 A
- US 2463264 A
- US 5237071 A
- US 5166355 A
- GB 2303549 A
- DE 19726184
- EP 893119 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- WO 04006878 A
- WO 05058269 A
- WO 06032741 A

- US 6225467 B
- WO 2004085412 A
- WO 06035000 A
- WO 06034982 A
- WO 06034991 A
- WO 06035007 A
- WO 2006034992 A
- WO 2006034985 A
- FR 2709666
- FR 2725369
- JP 2295912 A
- US 5412004 A
- US 5811487 A
- FR 2760641
- FR 2853543
- FR 2819175
- FR 2802425
- FR 2810548
- FR 2796278
- FR 2802420
- FR 2812544 A
- FR 2814950 A
- WO 0194381 A
- FR 2877220
- FR 2722380
- WO 0103538 A

**Littérature non-brevet citée dans la description**

- *Cosmetics & Toiletries,* Février 1990, vol. 105, 53-64
- **Jacques LE COZ.** Traité de mésothérapie. Masson, 2004

- **Bell E. et al.** The reconstitution of living skin. *J Invest Dermatol,* Juillet 1983, 81
- **Asselineau et al.** Models in Dermato. 1987, vol. III, 1-7